(19) Europäisches Patentamt
European Patent Office
Office européen des brevets

(11) **EP 4 664 405 A1**

(12) **EUROPEAN PATENT APPLICATION**

(43) Date of publication:
**17.12.2025 Bulletin 2025/51**

(21) Application number: **24181014.2**

(22) Date of filing: **10.06.2024**

(51) International Patent Classification (IPC):
**G06T 13/20** (2011.01)     **G06T 19/20** (2011.01)

(52) Cooperative Patent Classification (CPC):
**G06T 13/20;** G06T 19/20; G06T 2210/41;
G06T 2210/44; G06T 2210/62; G06T 2219/2021

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **3Shape A/S
1060 Copenhagen K (DK)**

(72) Inventors:
• **HUSEINI, Admir
  1060 Copenhagen K (DK)**
• **VETERE, Elmiro
  1060 Copenhagen K (DK)**
• **STOUSTRUP, Asger
  1060 Copenhagen K (DK)**
• **KURALT, Marko
  1060 Copenhagen K (DK)**

(74) Representative: **Guardian
IP Consulting I/S
Diplomvej, Building 381
2800 Kgs. Lyngby (DK)**

(54) **METHOD AND SYSTEM FOR PROCESSING 3D MODELS OF A DENTAL SITUATION**

(57) A computer-implemented method for processing digital 3D models of a dental situation is disclosed. The method comprises receiving, in a common 3D space, a first digital 3D model (100) representative of the dental situation at a first time, and a second digital 3D model (101) representative of the dental situation at a second time, the second time being later than the first time. The method further comprises displaying a transition of a tooth (102) of the first digital 3D model (100) into a corresponding tooth (104) of the second digital 3D model (101), wherein the displaying comprises aligning the tooth (102) and the corresponding tooth (104) at an intermediate position between the tooth (102) and the corresponding tooth (104), generating an adapter tooth (301) based on the shape of the tooth (102) and the shape of the corresponding tooth (104), wherein the shape of the adapter tooth (301) is a weighted average of the shape of the tooth (102) and the shape of the corresponding tooth (104), mapping the adapter tooth (301) to the tooth (102) to obtain the adapter tooth at a source position, and mapping the adapter tooth (301) to the corresponding tooth (104) to obtain the adapter tooth at a destination position. Displaying further comprises applying a first transparency transition from the tooth (102) to the adapter tooth at the source position, displacing the vertices of the adapter tooth at the source position to the adapter tooth at the destination position, applying a second transparency transition from the adapter tooth at the destination position to the corresponding tooth (104).

FIG. 6

EP 4 664 405 A1

**Description**

**Technical field**

The disclosure relates to a computer-implemented method and system for morphing of digital three-dimensional (3D) models of a dental situation. In particular, the disclosure relates to a computer-implemented method and system for modelling and displaying a transition of a tooth of a first digital 3D model into a corresponding tooth of a second digital 3D model.

**Background**

Development of intraoral scanning techniques has been instrumental in the transition to modern digital dentistry. Use of intraoral 3D scanners allows dental practitioners to accurately and quickly capture dental situation of a patient, which may then be visualized on a display as a digital three-dimensional (3D) model. Obtained digital 3D model may thus serve as a digital impression of teeth, offering numerous advantages over a classical physical impression of teeth.

Scanning of the dental situation of the same patient over a course of time may result in a series of digital 3D models which may be compared to each other for, for example, diagnostic purposes, to detect and visualize presence of a dental condition such as plaque, caries, gingivitis, dental recession and/or tooth wear. Such generated series of digital 3D models does not show how the dental situation is likely changing over the time but only offers snapshots of the dental situation in several time instances.

There is a need to develop methods and systems to enable conveying the clinically relevant information, about how the dental situation is changing, both to the dental practitioner and to the patient. Currently available methods of morphing directly the digital 3D models of teeth one into another have been proven challenging if there are significant geometric changes between the digital 3D models.

The present disclosure addresses prospects to utilize the capabilities of the intraoral 3D scanner systems to generate and display a smooth transition of the series of digital 3D models and thereby to detect and visualize clinical changes amongst them, even in the case of significant geometric changes.

**Summary**

In an embodiment, a computer-implemented method for morphing digital 3D models of a dental situation is disclosed, wherein the method comprises:

- receiving, in a common 3D space, a first digital 3D model representative of the dental situation at a first time, and a second digital 3D model representative of the dental situation at at a second time, the second time being later than the first time,
- displaying a transition of a tooth of the first digital 3D model into a corresponding tooth of the second digital 3D model, wherein the displaying comprises:
- aligning the tooth and the corresponding tooth at an intermediate position between the tooth and the corresponding tooth,
- generating an adapter tooth based on the shape of the tooth and the shape of the corresponding tooth, wherein the shape of the adapter tooth is a weighted average of the shape of the tooth and the shape of the corresponding tooth,
- mapping the adapter tooth to the tooth to obtain the adapter tooth at a source position, and mapping the adapter tooth to the corresponding tooth to obtain the adapter tooth at a destination position,
- applying a first transparency transition from the tooth to the adapter tooth at the source position,
- displacing the vertices of the adapter tooth at the source position to the adapter tooth at the destination position,
- applying a second transparency transition from the adapter tooth at the destination position to the corresponding tooth.

In a further embodiment, a computer-implemented method for morphing digital 3D models of the dental situation is disclosed, wherein the method comprises:

- receiving, in the common 3D space, the first digital 3D model representative of the dental situation at the first time, and the second digital 3D model representative of the dental situation at the second time, the second time being later than the first time,
- aligning the tooth of the first digital 3D model and the corresponding tooth of the second digital 3D model at the intermediate position between the tooth and the corresponding tooth,
- generating the adapter tooth based on the shape of the tooth and the shape of the corresponding tooth, wherein the shape of the adapter tooth is the weighted average of the shape of the tooth and the shape of the corresponding tooth,
- mapping the adapter tooth to the tooth to obtain the adapter tooth at the source position, and mapping the adapter tooth to the corresponding tooth to obtain the adapter tooth at the destination position,
- applying the first transparency transition from the tooth to the adapter tooth at the source position,
- displacing the vertices of the adapter tooth at the source position to the adapter tooth at the destination position,
- applying the second transparency transition from the adapter tooth at the destination position to the corresponding tooth.

An advantage of the method according to the disclosure is that a realistic, resource-efficient simulation of changes in the dental situation may be generated and conveyed to the user.

Expression "3D" throughout the present disclosure refers to a term "three-dimensional". Term "digital 3D model of a dental situation" refers to a digital, three-dimensional, computer-generated representation of the patient's dental situation.

Such digital 3D model may accurately correspond to the actual dental situation. That means that dental objects like teeth, teeth surfaces, restorations and/or gingiva on the digital 3D model may correspond to those of the dental situation.

The digital 3D model may be constructed by a processor, based on scan data collected in an intraoral scanning process in which an intraoral 3D scanner may be used to scan the patient's dental situation comprising teeth and gingiva. The intraoral 3D scanner throughout the disclosure is also referred to as the intraoral scanner. The digital 3D model can be stored in a memory of a computer system, for example in a Standard Triangle Language (STL) format.

The digital 3D model can be received or accessed by the processor. The digital 3D model may usually be displayed on a display screen in form of a 3D mesh, representing surfaces of teeth and gingival tissue of the dental situation. The 3D mesh may be comprised of individual facets, for example triangular facets while each facet may comprise, for example, three mutually connected vertices. Alternatively, the digital 3D model may be displayed as a point cloud comprising points, a graph comprising nodes and edges, a volumetric representation comprising voxels, or any other suitable 3D representation form.

The method according to an embodiment may comprise receiving, in the common 3D space, the first digital 3D model representative of the dental situation at the first time, and receiving the second digital 3D model representative of the dental situation at the second time, the second time being later than the first time. Thus, the second digital 3D model may comprise differences with respect to the first digital 3D model, those differences representing changes that have occurred in the dental situation during the time elapsed from the first time to the second time. Such changes may reflect clinically relevant changes in the dental situation such as development and/or progress of a dental condition.

The common 3D space may relate to a digital common 3D space presented to a user of the system. The first and second digital 3D models may be received, by the processor, in the common 3D space as well as visualized (rendered) in the common 3D space.

The tooth may be a 3D mesh representing both a tooth and its surrounding gingiva.

In another embodiment of the disclosure, a computer-implemented method for morphing digital 3D models of the dental situation is disclosed, wherein the method comprises:

- receiving, in the common 3D space, the first digital 3D model representative of the dental situation at the first time, and the second digital 3D model representative of the dental situation at at the second time, the second time being later than the first time,
- displaying the transition of a dental region of the first digital 3D model into a corresponding dental region of the second digital 3D model, wherein the displaying comprises:
- aligning the dental region and the corresponding dental region at the intermediate position between the tooth and the corresponding tooth,
- generating an adapter dental region based on the shape of the dental region and the shape of the corresponding dental region, wherein the shape of the adapter dental region is the weighted average of the shape of the dental region and the shape of the corresponding dental region,
- mapping the adapter dental region to the dental region to obtain the adapter dental region at the source position, and mapping the adapter dental region to the corresponding dental region to obtain the adapter dental region at the destination position,
- displacing the vertices of the adapter dental region at the source position to the adapter dental region at the destination position.

The dental region in this case may comprise a tooth and its surrounding gingiva while the corresponding dental region may comprise the corresponding tooth and its surrounding gingiva.

The method may further comprise displaying the transition of the tooth of the first digital 3D model into the corresponding tooth of the second digital 3D model. In this way the process of change in the morphology of the tooth may be highlighted to a user. The aim of the method of the disclosure is to display the transition in a smooth, gradual fashion in which the tooth is morphed into the corresponding tooth. This transition is a continuous transition showing how the tooth and/or gingiva change over time. This continuous transition credibly assists the dental professional to detect the change, quantify the change, detect rate of change, assess the patterns of how the change occurs, all of which are relevant clinical information. The dental professional may, based on the displayed transition of teeth and gingiva change, plan for orthodontic treatment. The displaying may occur on a display such as that of a computer device.

Throughout the disclosure, the corresponding tooth may be understood as a tooth on the second digital 3D model with the same dental notation UNN number (Universal Numbering System) as the tooth on the first digital 3D model. For example, tooth with UNN = 2 on the first digital 3D model has a corresponding tooth, on the second digital 3D model, also with UNN = 2.

A common practice to model the transition of the tooth into the corresponding tooth may be to morph the geometry of the tooth directly into the geometry of the corresponding tooth. However, such approach may be challenging if there is a significant change in two geometries. For example, if the tooth comprises braces and the corresponding tooth does not, then it may be particularly difficult to identify corresponding points of the tooth and the corresponding tooth, and to perform direct geometry morphing. A further example of a significant change is if a tooth is removed and an implant screw is inserted. Additionally, morphing of the interproximal ares between the neighboring teeth is technically challenging. In addition, the direct geometry morphing may be computationally expensive. In general, it can be said that modelling of changes using original 3D models does not offer required flexibility, it is computationally expensive and the end results are often incorrectly morphed shapes. The present disclosure however offers a technical solution overcoming the stated challenges.

In an example of the disclosure, displaying the transition of the tooth of the first digital 3D model into a corresponding tooth of the second digital 3D model may comprise aligning the tooth and the corresponding tooth at the intermediate position between the tooth and the corresponding tooth.

Aligning the tooth and the corresponding tooth at the intermediate position may comprise determining, and applying, a tooth rigid transformation that aligns the tooth with the corresponding tooth. Generally, a rigid transformation is comprised of a rotation and a translation that allow alignment of two 3D objects.

Aligning the tooth and the corresponding tooth at the intermediate position may comprise applying a first rigid transformation to the tooth to obtain an intermediate tooth and applying a second rigid transformation to the corresponding tooth to obtain an intermediate corresponding tooth, wherein the first rigid transformation and the second rigid transformation together define the tooth rigid transformation between the tooth and the corresponding tooth. Thus, the first rigid transformation and the second rigid transformations may be portions of the tooth rigid transformation determined for the tooth and the corresponding tooth.

Intermediate tooth and the intermediate corresponding tooth may together be referred to as intermediate meshes. The aim of generating these intermediate meshes is to model rigid tooth movement of the tooth, and these meshes may optionally be used in the method according to an embodiment.

The first rigid transformation may be in range of 300-700 of the tooth rigid transformation determined for the tooth and the corresponding tooth. Preferably, the first rigid transformation is 50% of the tooth rigid transformation.

For example, the first rigid transformation may be 550 of the tooth rigid transformation and the second rigid transformation may be 450 of the tooth rigid transformation. This means that the rotation of the first rigid transformation is 550 of the rotation of the tooth rigid transformation, and the translation of the first rigid transformation is 550 of the translation of the tooth rigid transformation. The positive effect of balancing out imperfections of 3D objects due to scanning technique variation is highest when the first and second half of the alignment rigid transformation are equal or close to equal.

In an example, aligning the tooth and the corresponding tooth at the intermediate position may comprise applying half of the tooth rigid transformation to the tooth and applying half of the tooth rigid transformation to the corresponding tooth. That may be understod as that the tooth of the first digital 3D model would be rotated and translated by half of the amount needed to fully align the tooth with the corresponding tooth. Similarly, the corresponding tooth of the second digital 3D model would be rotated and translated equally by half of the amount of the tooth rigid transformation.

The intermediate position may be a position along the translation path of the tooth rigid transformation.

In an embodiment, aligning the tooth and the corresponding tooth at the intermediate position may furter comprise interpolating an interproximal teeth area between the corresponding tooth and its neighboring tooth.

Interpolating the interproximal teeth area between the corresponding tooth and its neighboring tooth may be performed by utilizing Dijkstra algorithm.

Moving the tooth and the corresponding tooth by half of the tooth rigid transformation may be performed to obtain a starting point for the morphing in which the imperfections of the tooth and the corresponding tooth, caused by different scan quality, are balanced out. A further advantage to this alignment step is reflected in minimized amount of interpolation needed for the teeth interproximal area and gingival area.

Prior to aligning the tooth with its corresponding tooth, the first digital 3D model and the second digital 3D model may optionally be globally aligned. Generally, the first and second digital 3D models may occupy arbitrary positions in the common 3D space. Global alignment of said two digital 3D models may comprise determining a full rigid transformation that transforms the first digital 3D model into the second digital 3D model. An advantage of having this global alignment of complete digital 3D models is reflected in that a better starting position is obtained for the following alignment on an individual tooth level.

The full rigid transformation, consisting of a rotation and a translation, may be determined, which describes how the first and/or the second digital 3D models should be moved in the common 3D space, in order to align the two digital 3D models. Alignment may refer to overlapping the two digital 3D models.

Alignment may be performed such that all of the teeth of the first digital 3D model are aligned to their corresponding teeth of the second digital 3D model. In general, corresponding teeth may be the teeth with the same dental notation UNN (Universal Numbering System) number. In addition or alternatively to the alignment of teeth, gingiva of the first digital 3D model may be aligned with the gingiva of the second digital 3D model.

In an example, any alignment step of the disclosure may be performed based on Iterative Closest Point (ICP) principle. Alternatively, alignment may be done manually, for example by identifying at least three common points on the respective objects.

Displaying the transition of the tooth of the first digital 3D model into the corresponding tooth of the second digital 3D model may further comprise generating the adapter tooth based on the shape of the tooth and the shape of the corresponding tooth, wherein the shape of the adapter tooth is the weighted average of the shape of the tooth and the shape of the corresponding tooth. In this way a shape similar to both the tooth and the corresponding tooth may be obtained which can then be utilizied for simulating the transition.

The adapter tooth may be a digital 3D model shape of which is the weighted average of the shape of the tooth and the shape of the corresponding tooth.

In an embodiment, the shape of the adapter tooth may be 500 of the shape of the tooth and 50% the shape of the corresponding tooth. In this way a shape may be obtained that is similar both to the tooth and the corresponding tooth as it is the weighted average of the two.

The role of the adapter tooth may be to connect the two tooth meshes that are not initially compatible, for example the tooth and the corresponding tooth when one has braces. The adapter tooth thereby may serve as an intermediate tooth, geometry of which can be adapted to both teeth meshes. The adapter tooth may be used to model the transition from the tooth to the corresponding tooth, where the transition comprises shape change and/or tooth movement change.

The adapter tooth may also be referred to as an adapter mesh, as it is a 3D mesh that can be in form of e.g. a tooth. In an example, generating the adapter tooth may comprise applying a signed distance field algorithm to the tooth and to the corresponding tooth, to obtain a functional representation of the tooth and a functional representation of the corresponding tooth.

Generating the adapter tooth may further comprise obtaining an interpolation function of both the functional representation of the tooth and the functional representation of the corresponding tooth.

Generating the adapter tooth may further comprise applying a marching cubes algorithm to the obtained interpolation function. As a result, a digital 3D mesh of the adapter tooth may be obtained.

Mapping the adapter tooth to the tooth may comprise identifying, from each vertex of the adapter tooth, a corresponding closest point on the tooth, and displacing the each vertex of the adapter tooth to the identified corresponding closest point on the tooth. Mapping the adapter tooth to the corresponding tooth may comprise identifying, from each vertex of the adapter tooth, a corresponding closest point on the corresponding tooth, and displacing the each vertex of the adapter tooth to the identified corresponding closest point on the corresponding tooth. The closest points may be identified using the Itterative Closest Point (ICP) algorithm.

The method according to the disclosure may comprise applying the first transparency transition from the tooth to the adapter tooth at the source position. This means that the transparency of the tooth and the transparency of the adapter tooth are gradually varied such that the tooth becomes invisible while the adapter tooth becomes visible instead.

The method may further comprise displacing the vertices of the adapter tooth at the source position to the adapter tooth at the destination position. This step represents modelling of geometry change between two digital 3D objects of interest.

The method may additionally comprise applying the second transparency transition from the adapter tooth at the destination position to the corresponding tooth. This means that the transparency of the tooth and the transparency of the adapter tooth are gradually varied such that the adapter tooth becomes invisible while the corresponding tooth becomes visible instead.

An advantage of the methods presented is that a continuous and realistic simulation of dental changes can be modelled and presented, by combining the transparency transitions with the displacement of adapter tooth vertices. Additionally, such obtained simulation is computationally efficient compared to known alternative methods.

In the method according to the disclosure, displaying the transition of the tooth into the corresponding tooth may comprise simulating the change between the tooth and the corresponding tooth. In one example, the change may be a change in tooth morphology and/or change in tooth movement. In yet a further example, the change may be a change in severity of a detected dental condition.

The dental condition may be any one or more of tooth wear, plaque, gingivitis, caries and/or recession.

In an embodiment, the change between the tooth and the corresponding tooth may comprise tooth movement. For example, the corresponding tooth may move to a different position relative to the position of the tooth. Displaying the transition of the tooth into the corresponding tooth may comprise indicating this direction of tooth movement. Additionally or alternatively, the change may comprise a change in the dental condition, such as a severity of the dental condition. Displaying the transition of the tooth into the corresponding tooth may thus comprise indicating a direction of progress of the dental condition.

The dental condition may be tooth wear in an example. The method may comprise determining the amount of tooth wear on the corresponding tooth relative to the tooth by determining a distance between corresponding facets of the tooth and the corresponding tooth.

Additionally, processing of the digital 3D model may comprise detecting of the dental condition present in the dental situation.

In the method according to an embodiment, the method may further comprise identifying a region on the tooth and/or the corresponding tooth corresponding to the difference between the tooth and the corresponding tooth. The region may be a surface region comprised of one or more facets of the the tooth and/or the corresponding tooth. The region corresponding to the difference between the tooth and the corresponding tooth may be identified, for example, by direct comparison of geometries of the the tooth and the corresponding tooth. The region may also be referred to as an area of interest.

Furthermore, the method of the disclosure may comprise determining a volumetric difference of the identified region. In this way a volumetric measurement of the change that has occurred may be determined. The determined volumetric difference of the region may be displayed, for example on the computer display.

The identified region may, according to an embodiment, be highlighted. Highlighting of the region may comprise, in an example, coloring the region with outline or filled area. Alternatively or additionally, highlighting the region may comprise applying a pulsing effect on the region or displaying an alert sign.

The method according to an embodiment may further comprise determining a presence of the dental condition in the identified region. The presence of the dental condition may be established, for example, by comparing values of vertice surface features to established thresholds. The surface features of a vertice may be any one or more of a curvature, a position, a surface normal. Additionally, color values of vertices may be compared to color value thresholds to determine presence of the dental condition, for example caries. Alternatively, the presence of the dental condition may be established by applying a trained neural network to the tooth and/or the corresponding tooth.

Displaying the transition of the tooth into the corresponding tooth, in an example, may occur on the first and/or the second digital 3D model. In this way the user may directly observe the transition at the corresponding area of the first and/or second digital 3D model. Alternatively, displaying the transition can occur in a window separate of the first and/or the second 3D dental model. The window may be a magnifying glass.

Displaying the transition may occur in response to a user's interaction with the identified region such as hovering of a pointer over the region.

The method according to an embodiment may further comprise:

- predicting a future state of the tooth, and
- displaying the transition of the corresponding tooth into the future state of the tooth.

In this way an extrapolation into the future predicted state of the tooth is done, allowing the user to visually observe progress of the change, such as the progress of the dental condition.

In an example, the future state of the tooth may be a prediction of an orthodontic treatment outcome where the future state of the tooth represents the tooth moved into a desired position in the jaw. Alternatively or additionally, the future state of the tooth may be a prediction of a tooth bleaching treatment where the future state of the tooth corresponds to a desired color, different to the color of the tooth. Alternatively or additionally, the future state of the tooth may be a prediction of the dental condition development. For example, the future state of the tooth may reflect further progress of tooth wear following rate of tooth wear previously detected.

In an example, the future state of the tooth may be predicted by linearly extrapolating, from the corresponding tooth, the previously obtained transition of the tooth into the corresponding tooth. For example, same amount of vertice displacement may be furter applied to the adapter tooth at the destination position as previously applied to displace the vertices of the adapter tooth at the source position to the adapter tooth at the destination position. In that way, a geometry of the future state of the tooth may be obtained.

This prediction of the future state of the tooth may be useful as the dentist may visualize, to the patient, the possible consequence of keeping poor dental hygiene habits or the possible outcome of a dental orthodontic treatment.

The method of an embodiment may further comprise sending a recording of the transition of the tooth into the corresponding tooth to an external viewing device. The method may alternatively or additionally comprise uploading the recording of the transition of the tooth into the corresponding tooth or a future state of the tooth to a cloud storage device.

So far, the method of the disclosure was presented with respect to two digital 3D models of the dental situation. However, the method is applicable also for more than just two digital 3D models. A series of digital 3D models may exist representing the dental situation over a course of time.

Thereby, the method according to an example may further comprise:

- receiving, in the common 3D space, a third digital 3D model representative of the dental situation at a third time, the third time being later than the second time, and
- further displaying a transition of the corresponding tooth into a further corresponding tooth of the third digital 3D model.

Displaying the transition of the corresponding tooth into the further corresponding tooth of the third digital 3D model may comprise:

- aligning the corresponding tooth and the further corresponding tooth at a further intermediate position between the corresponding tooth and the further corresponding tooth,
- generating a further adapter tooth based on the shape of the corresponding tooth and the shape of the further corresponding tooth, wherein the shape of the further adapter tooth is a weighted average of the shape of the corresponding tooth and the shape of the further corresponding tooth,
- mapping the further adapter tooth to the corresponding tooth to obtain the further adapter tooth at a further source position, and mapping the further adapter tooth to the further corresponding tooth to obtain the further adapter tooth at a further destination position,
- applying a third transparency transition from the corresponding tooth to the further adapter tooth at the further source position,
- displacing the vertices of the further adapter tooth at the further source position to the further adapter tooth at the further destination position,
- applying a fourth transparency position from the furter adapter tooth at the further destination position to the further corresponding tooth.

According to the disclosure, a computer-implemented method for processing digital 3D models of the dental situation is disclosed, the method comprising:

- receiving, in the common 3D space, a plurality of digital 3D models representative of the dental situation at different times,
- displaying a transition of a tooth through each of the plurality of the digital 3D models, wherein the displaying comprises:
- aligning the tooth with all of its corresponding teeth of the plurality of digital 3D models at an intermediate position,
- generating the adapter tooth based on the shape of the tooth and the shape of all of its corresponding teeth, wherein the shape of the adapter tooth is a weighted average of the shapes of the tooth and all of its corresponding teeth,
- mapping the adapter tooth to the tooth to obtain the adapter tooth at the source position, and mapping the adapter tooth to all of its corresponding teeth to obtain the adapter tooth at a plurality of destination positions,
- displacing the vertices of the adapter tooth at the source position to the adapter tooth at each of the plurality of destination positions.

The method according to an embodiment of the disclosure may further comprise starting and/or pausing of displaying the transition based on the user interaction with a user interface.

The method may further comprise indicating the progress of the transition on a timeline.

The method may further comprise, based on user interaction with the timeline, modifying the progress of the transition. In this way the user can fast-forward and/or rewind through the simulation.

The method may further comprise, based on user interaction with the timeline, interrupting the transition and displaying a digital 3D model.

The method according to an example may further comprise, based on user interaction with the user interface, repeating/rewinding the transition once the transition reaches the end.

According to the disclosure a dental scanning system is disclosed, wherein the dental scanning system comprises:

- a display device for displaying the transition of the tooth of the first digital 3D model into the corresponding tooth of the second digital 3D model,
- a processor configured to perform the steps of any of the previously disclosed methods.

A computer program product is further disclosed, the computer program product comprising instructions which, when executed by a computer, cause the computer to perform the method according to any of the embodiments presented herein.

**Brief description of the figures**

FIG. 1 illustrates a first digital 3D model of the dental situation and the second digital 3D model of the dental situation;

FIG. 2 illustrates steps of the method according to an embodiment;

FIG. 3 is a flowchart illustrating generation of the adapter tooth;

FIG. 4 illustrates steps of the first and second transparency transition utilized by the method according to an embodiment;

FIG. 5 illustrates a dental scanning system capable of performing the method according to an embodiment the disclosure.

FIG. 6 illustrates a graphical user interface displaying the transition of the first digital 3D model into the second digital 3D model.

**Detailed description**

In the following description, reference is made to the accompanying figures, which show by way of illustration how the invention may be practiced.

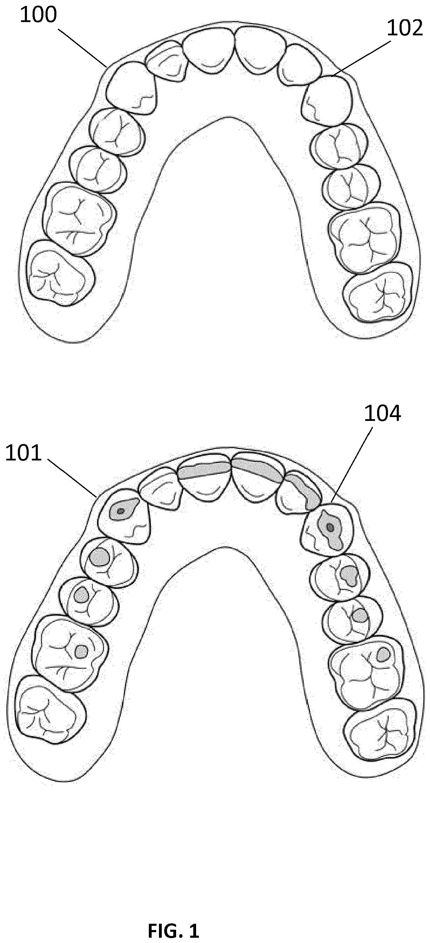

FIG. 1 illustrates a first digital three-dimensional (3D) model 100 of patient's dental situation and a second three-dimensional (3D) model 101 of the same dental situation. The first digital three-dimensional model 100 may be representative of the patient's teeth at a first time such as a first visit to a dental clinic, while the second digital three-dimensional model 101 may be representative of the patient's teeth at a second time such as a second visit to a dental clininc, the second time being later than the first time.

The first digital 3D model 100 and the second digital 3D model 101 may be different. The differences may be reflected in the movement of certain teeth in between the first visit and the second visit to the dental clinic, and/or in the appearance and/or progress of a dental condition. Such dental condition may be, for example, tooth wear, illustrated as dark areas on the second three-dimensional model 101. These dark areas represent a loss in tooth material due to tooth wear. Thus, a difference in geometry between the first and second digital three-dimensional models 100, 101 may exist. The difference in geometry may be a result of change in morphology of teeth due to tooth wear. The difference may be, not indicated in FIG. 1, reflected in movement of teeth from the first digital 3D model 100 to the second digital 3D model 101. FIG. 1 illustrates the patient's upper jaw, however the first and/or second digital three-dimensional models 100, 101 may comprise additionally or alternatively the lower jaw or both the upper and the lower jaw.

For the interpretation of changes in the teeth of the patient, it may be desired to simulate how teeth and/or gingiva of the first digital 3D model 100 transition into the teeth and/or gingiva of the second digital 3D model 101. This transitioning may comprise tooth movement, change in tooth morphology, change in tooth color and/or tooth removal/disappearance. This simulated transition may be required for diagnostic purposes, such as for determining a direction of progression of dental conditions such as tooth wear, caries, dental plaque, tooth cracks, gingivitis and/or dental recession. Simulating the changes between the two digital 3D models may be required to track progress of an orthodontic treatment, to plan an orthodontic treatment and/or to plan for other dental procedures. Additionally, ability to simulate how changes occur may have a positive effect on patient engagement and acceptance to dental treatments as treatments may be effectively explained.

Simulating the changes that occur in tooth and/or gingiva tissue may also be advantageous for quantifying and presenting the amount of change to the user. Compared to conventional use of color maps that indicate severity of change, simulating the transition of a tooth from its past state to present or future state offers a much simpler and clearer solution.

The first and second digital three-dimensional models 100, 101 may have pairs of corresponding teeth.

Corresponding teeth may relate to teeth with the same Universal Numbering System notation (UNN) on the first and second digital three-dimensional model 100, 101. For example, the canine 102 on the first digital three-dimensional model 100 has a corresponding canine 104 on the second digital three-dimensional model 101 with the same UNN notation. Thereby, tooth 102 and its corresponding tooth 104 illustrated in FIG. 1 are an example of corresponding teeth.

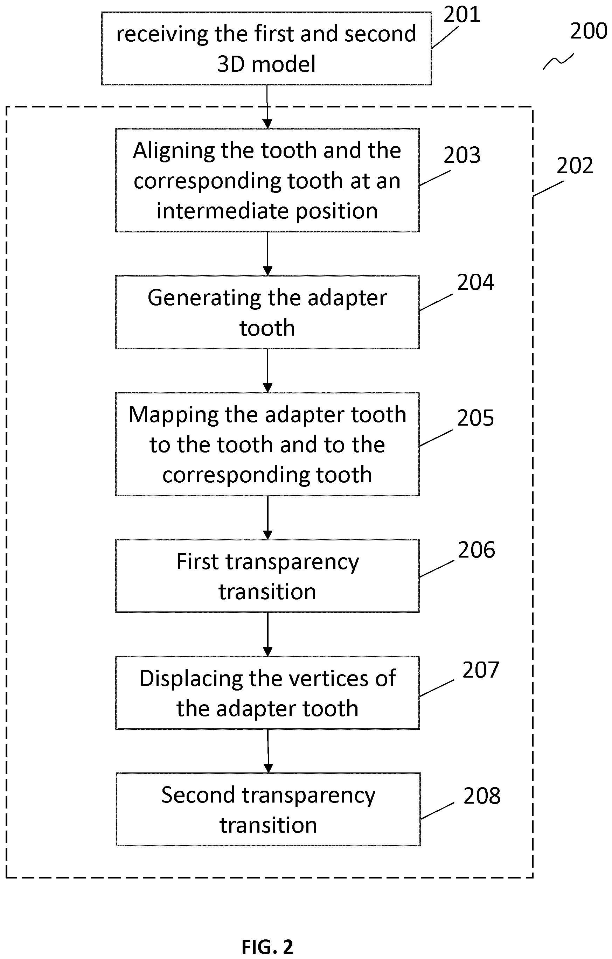

FIG. 2 illustrates a flowchart of a method 200 according to the disclosure. In step 201 the first digital 3D model 100 of the dental situation and the second digital 3D model 101 of the dental situation may be received by the processor. The first digital 3D model 100 may comprise a plurality of teeth while the second digital 3D model 101 may comprise a plurality of corresponding teeth with same UNN numbers. The corresponding teeth of the second digital 3D model 101 may have changed, in their position, shape and/or color compared to the plurality of teeth in the first digital 3D model 100. Additionally, gingiva of the second digital 3D model 101 may have changed compared to gingiva of the first digital 3D model 100, for example due to gingivitis or dental recession.

The digital 3D models 100, 101 may be segmented such that individual dental objects such as teeth and gingiva can be treated separately. The segmentation process may be performed in different ways, for example based on identification of individual facet or group of facets belonging to a tooth or gingiva. It thus allows for identifying objects such as individual teeth and/or surrounding gingiva in the digital 3D models representing the patient's dentition. Therefore, the output of the segmentation process may be individual tooth representations which are usually displayed in form of solid tooth objects, tooth meshes or tooth point clouds.

In the method according to the embodiment a transition of the tooth 102 of the first digital 3D model 100 into the corresponding tooth 104 of the second digital 3D model 101 may be displayed, in order to visualize the movement and/or morphological change of the tooth 102 over time. This may also be referred to as morphing or simulation of tooth and/or gingiva change and is indicated with numeral 202 in FIG. 2.

Although the method steps in the following paragraphs focus on a single tooth-pair, the steps are applicable to other tooth-pairs and/or gingiva that may be of interest from the point of view of morphology, color and/or movement change.

Displaying the transition may comprise, in step 203, aligning the tooth 102 and the corresponding tooth 104 at an intermediate position between the tooth 102 and the corresponding tooth 104. This may be performed by calculating a tooth rigid transformation that aligns the tooth pair, namely the tooth 102 with the corresponding tooth 104. The tooth rigid transformation may comprise a rotation and a translation parameter defining how an amount of translation and rotation necessary for aligning the tooth 102 with the corresponding tooth 104. Subsequently, vertices of the tooth 102 may be moved towards the corresponding tooth 104, and vertices of the corresponding tooth 104 may be moved towards the tooth 102 by applying the determined tooth rigid transformation to an extent such that the intermediate position is reached. The intermediate position may lie along the translation path between the tooth 102 and the corresponding tooth 104.

Preferably, the intermediate position is obtained by applying 500 of the calculated tooth rigid transformation to both the tooth 102 and the corresponding tooth 104. The positive effect of balancing out imperfections of 3D objects due to scanning technique variation may be achieved when the tooth 102 and the corresponding tooth 104 are moved equally or substantially equally.

In an example, aligning the tooth 102 and the corresponding tooth 104 at the intermediate position may comprise applying half of the tooth rigid transformation to the tooth 102 and applying half of the tooth rigid transformation to the corresponding tooth 104. That means that the tooth 102 of the first digital 3D model 100 may be rotated and/or translated by half of the amount needed to fully align the tooth 102 with the corresponding tooth 104. Similarly, the corresponding tooth 104 of the second digital 3D model 101 may be rotated and/or translated equally by half of the amount of the tooth rigid transformation. By aligning the tooth 102 and the corresponding tooth 104 in this manner, movement of the tooth 102 may be simulated.

By performing the said alignment, intermediate meshes may be obtained. Aligning the tooth 102 and the corresponding tooth 104 at the intermediate position may comprise applying a first rigid transformation to the tooth 102 to obtain an intermediate tooth and applying a second rigid transformation to the corresponding tooth 104 to obtain an intermediate corresponding tooth, wherein the first rigid transformation and the second rigid transformation together define the tooth rigid transformation between the tooth 102 and the corresponding tooth 104. Thus, the first rigid transformation and the second rigid transformations are portions of the tooth rigid transformation determined for the tooth 102 and the corresponding tooth 104.

The intermediate tooth and the intermediate corresponding tooth may together be referred to as intermediate meshes. The aim of generating these intermediate meshes is to model rigid tooth movement of the tooth, and these meshes may optionally be used in the method according to an embodiment.

Method according to an embodiment may further comprise interpolating an interproximal teeth area between the tooth 102 and its neighboring tooth. This implies that the vertices of the interproximal teeth area may be moved together with the vertices of the tooth 102.

In an embodiment, the method may further comprise interpolating gingival area between the tooth 102 and its neighboring tooth. This implies that the vertices of the gingival area may be moved together with the vertices of the tooth 102.

Interpolating the interproximal teeth area and/or gingival area between the tooth 102 and its neighboring tooth may be based on applying the Dijkstra algorithm.

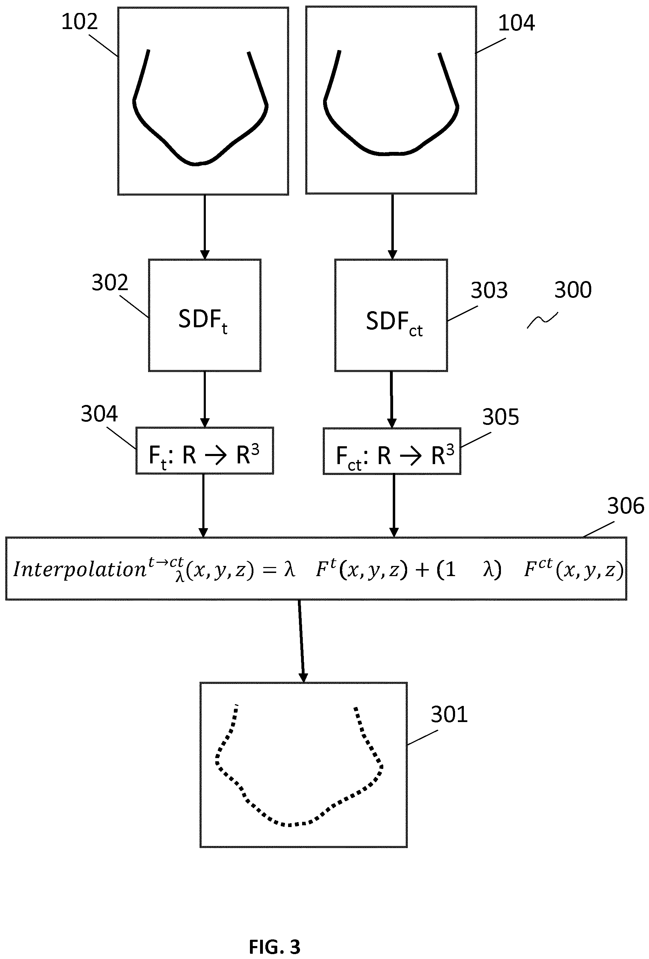

In the step 204 an adapter tooth 301 may be generated based on the shape of the tooth 102 and the shape of the corresponding tooth 104. The adapter tooth 301 may be a three-dimensional mesh whose shape is a weighted average of the shape of the tooth 102 and the shape of the corresponding tooth 104. Process of obtaining the adapter tooth 301 is further elaborated with respect to FIG. 3.

The role of the adapter tooth 301 may be to connect the two tooth meshes that are not initially compatible, for example the tooth 102 with the corresponding tooth 104 which has a different morphology. The adapter tooth 301 thereby may serve as an intermediate tooth, geometry of which can be adapted to both teeth meshes. The adapter tooth 301 may thus be used to model the transition from the tooth 102 to the corresponding tooth 104, wherein the transition comprises tooth morphology change.

In step 205 of the method 200 the adapter tooth 301 may be mapped to the tooth 102 and to the corresponding tooth 104. Mapping may be performed using Itterative Closest Point (ICP) algorithm such that, for each vertex of the adapter tooth 301, closest points are identified both on the tooth 102 and on the corresponding tooth 104. The vertices of the adapter tooth 301 may then be displaced to the identified closest points.

By mapping the adapter tooth 301 to the tooth 102, a source position for the adapter tooth 301 may be obtained. Similarly, by mapping the adapter tooth 301 to the corresponding tooth 104 a destination position for the adapter tooth 301 may be obtained. The vertices of the adapter tooth 301 may then be displaced from the source position to the destination position, thereby simulating the morphology change of the tooth 102.

An advantage of the method according to the disclosure is that the full three-dimensional geometry morphing from the tooth 102 into the corresponding tooth 104 does not need to be performed. Instead, the geometry of the adapter tooth 301 may be modified which greatly simplifies the transition of the tooth 102 into the corresponding tooth 104 in terms of computing resources and time required to perform the simulation.

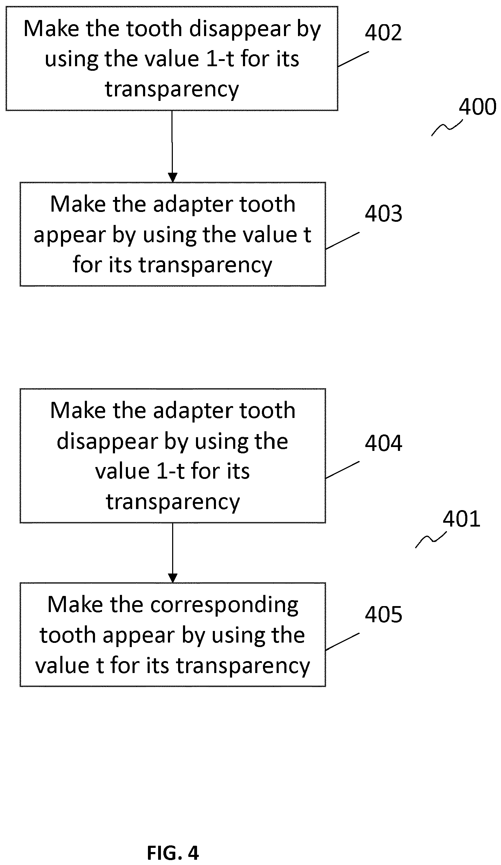

Prior to displacing the vertices of the adapter tooth 301, a first transparency transition may be performed, indicated with numeral 206 of the method 200. This transparency transition may be a 3D transparency modification with the aim of gradually "switching" from the tooth 102 to the adapter tooth 301 at the source position. In this way the tooth 102 may disappear and the adapter tooth 301 appears. This process is further illustrated in FIG. 4.

Step 207 of the method 200 illustrates displacing the vertices of the adapter tooth 301 at the source position to the adapter tooth 301 at the destination position.

After displacement of the vertices of the adapter tooth 301, a second transparency transition 208 may be performed, in order to gradually "switch" from the adapter tooth 301 at the destination position to the corresponding tooth 104.

The combination of the transparency transitions steps 206, 208, and the displacing of adapter tooth vertices step 207 represents an advantageous solution to efficiently model transition of the tooth 102 into the corresponding tooth 104.

FIG. 3 illustrates the process 300 of generating the adapter tooth 301. As mentioned before, shape of the adapter tooth 301 may be the weighted average of the shape of the tooth 102 and the corresponding tooth 104. FIG. 3 illustrates the tooth 102, the corresponding tooth 104 and the adapter tooth 301 as two-dimensional cross-sections, however they represent three-dimensional meshes.

The two 3D models 102, 104 are thus starting points for obtaining the adapter tooth 301. Blocks 302 and 303 illustrate generating Signed Distance Field (SDF) for the tooth ($SDF_t$) 102 and generating Signed Distance Field (SDF) for the corresponding tooth ($SDF_{ct}$) 104, respectively. The SDF algorithm assigns a numerical value to every point (x,y,z) on a 3D mesh where the numerical value can represent a distance from a boundary such as tooth contour. The SDF algorithm is known in the art and not discussed here in further detail.

The obtained Signed Distance Fields 302, 303 allow for deriving a functional representation of the tooth 304 and a functional representation of the corresponding tooth 305.

Next, an interpolation function 306 may be calculated for a desired value of coefficient $\lambda \in [0,1]$:

$$Interpolation^{t \to ct}_{\lambda}(x, y, z) = \lambda * F^t(x, y, z) + (1 - \lambda) * F^{ct}(x, y, z)$$

It can be noted that for a value of coefficient $\lambda=1$, the interpolation function 306 becomes the functional representation of the tooth 304. For a value of coefficient $\lambda=0$, the interpolation function 306 becomes the functional representation of the corresponding tooth 305. By applying intermediate values of coefficient A, between 0 and 1, the intermediate shapes between the tooth 102 and the corresponding tooth 104 may be obtained.

In the preferred embodiment the value of coefficient A may be selected as 0.5 to obtain the weighted average of the tooth 102 and the corresponding tooth 104.

The result of the interpolation function 306 may be transformed into the three-dimensional adapter tooth 301 by applying the Marching Cubes algorithm. This algorithm allows for extrapolating a 3D mesh from the interpolation function 306. An iso-surface may be generated by running the Marching Cubes algorithm on the determined Signed Distance Fields 302, 303. The generation of the iso-surface may be seen as the inverse operation of Signed Distance Field generation, recovering a 3D mesh from the obtained Signed Distance Fields. As this algorithm is known in the art, it is not discussed further in detail.

FIG. 4 illustrates flowcharts of the first transparency transition 400 and the second transparency transition 401. In step 402 parameter 1-t may be used to modify the transparency of the tooth 102, where t takes values from 0 to 1. This means that the transparency of the tooth 102 will eventually be modified such that the tooth 102 will not be visible. Step 403 illustrates varying transparency value t of the adapter tooth 301 at the source position in order to make the adapter tooth 301 appear once the tooth 102 has high transparency (disappears).

Similarly, FIG. 4 illustrates the second transparency transition 401 where parameter 1-t is applied to transparency of the adapter tooth 301 at the destination position to gradually make the adapter tooth 301 fade. In step 405, the corresponding tooth 104 may appear by applying a transparency value t from 0 to 1.

Both the first transparency transition 400 and the second transparency transition 401 may be performed on three-dimensional meshes.

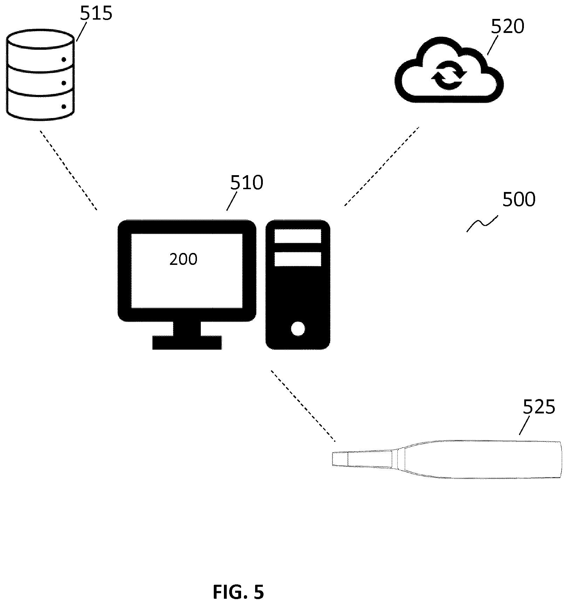

FIG. 5 illustrates a dental scanning system 500 which may comprise a computer 510 capable of carrying out any method of the disclosure. The computer may comprise a wired or a wireless interface to a server 515, a cloud server 520 and the intraoral scanner 525. The intraoral scanner 525 may be capable of recording the scan data comprising geometrical information, natural color information and/or fluorescence information associated with the patient's dentition. The intraoral scanner 525 may be equipped with various modules such as a fluorescence module or an infrared module and thereby capable of capturing information relevant for diagnosing dental conditions such as caries, dental recession, tooth cracks, gingivitis and/or plaque.

The dental scanning system 500 may comprise a data processing device configured to carry out the method according to one or more embodiments of the disclosure. The data processing device may be a part of the computer 510, the server 515 or the cloud server 520.

The dental scanning system 500 may comprise the data processing device configured to:

- receive, in a common 3D space, the first digital 3D model 100 representative of the dental situation at a first time, and the second digital 3D model 101 representative of the dental situation at a second time, the second time being later than the first time,
- display the transition of the tooth 102 of the first digital 3D model 100 into the corresponding tooth 104 of the second digital 3D model 101, wherein the displaying comprises:
- align the tooth 102 and the corresponding tooth 104 at the intermediate position between the tooth 102 and the corresponding tooth 104,
- generate the adapter tooth 301 based on the shape of the tooth 102 and the shape of the corresponding tooth 104, wherein the shape of the adapter tooth 301 is the weighted average of the shape of the tooth 102 and the shape of the corresponding tooth 104,
- map the adapter tooth 301 to the tooth 102 to obtain the adapter tooth at the source position, and mapping the adapter tooth 301 to the corresponding tooth 104 to obtain the adapter tooth at the destination position,
- apply the first transparency transition from the tooth 102 to the adapter tooth at the source position,
- displace the vertices of the adapter tooth at the source position to the adapter tooth at the destination position,
- apply the second transparency transition from the adapter tooth at the destination position to the corresponding tooth 104.

A non-transitory computer-readable storage medium may be comprised in the dental scanning system 500. The non-transitory computer-readable medium can carry instructions which, when executed by a computer, cause the computer to carry out the method according to one or more embodiments of the disclosure.

The non-transitory computer-readable medium can carry instructions which, when executed by a computer, cause the computer to:

- receive, in the common 3D space, the first digital 3D model 100 representative of the dental situation at a first time, and the second digital 3D model 101 representative of the dental situation at a second time, the second time being later than the first time,
- display the transition of the tooth 102 of the first digital 3D model 100 into the corresponding tooth 104 of the second digital 3D model 101, wherein the displaying comprises:
- align the tooth 102 and the corresponding tooth 104 at the intermediate position between the tooth 102 and the corresponding tooth 104,
- generate the adapter tooth 301 based on the shape of the tooth 102 and the shape of the corresponding tooth 104, wherein the shape of the adapter tooth 301 is the weighted average of the shape of the tooth 102 and the shape of the corresponding tooth 104,

- map the adapter tooth 301 to the tooth 102 to obtain the adapter tooth at the source position, and mapping the adapter tooth 301 to the corresponding tooth 104 to obtain the adapter tooth at the destination position,
- apply the first transparency transition from the tooth 102 to the adapter tooth at the source position,
- displace the vertices of the adapter tooth at the source position to the adapter tooth at the destination position,
- apply the second transparency transition from the adapter tooth at the destination position to the corresponding tooth 104.

A computer program product may be embodied in the non-transitory computer-readable storage medium. The computer program product may comprise instructions which, when executed by a computer, cause the computer to perform the method according to any of the embodiments presented herein.

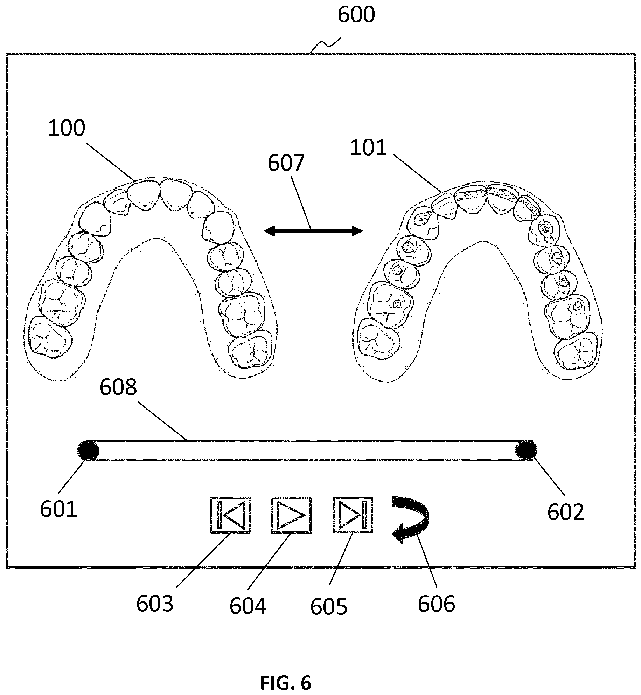

FIG. 6 illustrates a graphical user interface 600 displaying a transition of the first digital 3D model 100 into the second digital 3D model 101. The transition is illustrated with the arrow 607.

The graphical user interface 600 may comprise a bar 608 representing timeline. Points 601, 602 on the bar 601 may represent specific time points at which the dental situation has been scanned. For example, the point 601 may correspond to the first digital 3D model 100 while the point 602 may correspond to the second digital 3D model 101.

The graphical user interface 600 may be configured, based on user input, to display the transition. For example, by engaging a play button 604, the user may initiate the method for processing digital 3D models of the dental situation according to the disclosure.Similarly, the user may pause the displaying of the transition by engaging again the play button 604.

Buttons 603, 605 may be used by the user to move backward and forward in time, respectively, and in that way to skip the displaying of the transition.

Button 606 may be used by the user to rewind the displaying of the transition.

Progress of the transition may be displayed on the bar 608. Based on the user's interaction with the bar 608, the progress of the transition may be modified. For example, the user may advance or revert the progress by moving the points across the bar 608 or by clicking on a part of the bar 608.

It is to be understood that embodiments may be made, other than those mentioned, and structural and functional modifications may be made without departing from the scope of the present invention.

## Claims

**1.** A computer-implemented method for morphing digital 3D models of a dental situation, the method comprising:

- receiving, in a common 3D space, a first digital 3D model representative of the dental situation at a first time, and a second digital 3D model representative of the dental situation at a second time, the second time being later than the first time;
- displaying a transition of a tooth of the first digital 3D model into a corresponding tooth of the second digital 3D model, wherein the displaying comprises:
- aligning the tooth and the corresponding tooth at an intermediate position between the tooth and the corresponding tooth;
- generating an adapter tooth based on the shape of the tooth and the shape of the corresponding tooth, wherein the shape of the adapter tooth is a weighted average of the shape of the tooth and the shape of the corresponding tooth;
- mapping the adapter tooth to the tooth to obtain the adapter tooth at a source position, and mapping the adapter tooth to the corresponding tooth to obtain the adapter tooth at a destination position;
- applying a first transparency transition from the tooth to the adapter tooth at the source position;
- displacing the vertices of the adapter tooth at the source position to the adapter tooth at the destination position;
- applying a second transparency transition from the adapter tooth at the destination position to the corresponding tooth.

**2.** The method according to the previous claim 1, wherein aligning the tooth and the corresponding tooth at the intermediate position comprises determining a tooth rigid transformation that transforms the tooth into the corresponding tooth.

**3.** The method according to any previous claim, wherein aligning the tooth and the corresponding tooth at the intermediate position furter comprises interpolating an interproximal teeth area between the corresponding tooth and its neighboring tooth.

4. The method according to any previous claim, wherein the shape of the adapter tooth is 50% of the shape of the tooth and 500 the shape of the corresponding tooth.

5. The method according to any previous claim, wherein generating the adapter tooth comprises applying a signed distance field algorithm to the tooth and the corresponding tooth to obtain a functional representation of the tooth and a functional representation of the corresponding tooth.

6. The method according to the previous claim 5, wherein generating the adapter tooth further comprises obtaining an interpolation function of the functional representation of the tooth and the functional representation of the corresponding tooth.

7. The method according to the previous claim 6, wherein generating the adapter tooth further comprises applying a marching cubes algorithm to the obtained interpolation function.

8. The method according to any previous claim, wherein displaying the transition of the tooth of the first digital 3D model into the corresponding tooth of the second digital 3D model displays a simulated change between the tooth and the corresponding tooth.

9. The method according to the previous claim 8, wherein the change is a change in tooth morphology.

10. The method according to the previous claim 8, wherein the change is a change in a dental condition.

11. The method according to the previous claim 10, wherein the dental condition is tooth wear, further comprising determining the amount of tooth wear by determining a distance between corresponding facets of the tooth and the corresponding tooth.

12. The method according to any previous claim, further comprising:

   - predicting a future state of the tooth;
   - displaying the transition of the corresponding tooth into the future state of the tooth.

13. The method according to the previous claim 12, wherein the future state of the tooth is a prediction of an orthodontic treatment.

14. The method according to the previous claim 12, wherein the future state of the tooth is a prediction of a tooth bleaching treatment.

15. The method according to the previous claim 12, wherein the future state of the tooth is a prediction of the dental condition development.

**FIG. 1**

receiving the first and second 3D model — 201

200

Aligning the tooth and the corresponding tooth at an intermediate position — 203

202

Generating the adapter tooth — 204

Mapping the adapter tooth to the tooth and to the corresponding tooth — 205

First transparency transition — 206

Displacing the vertices of the adapter tooth — 207

Second transparency transition — 208

**FIG. 2**

$$Interpolation^{t \to ct}_{\lambda}(x, y, z) = \lambda \quad F^t(x, y, z) + (1 \quad \lambda) \quad F^{ct}(x, y, z)$$

**FIG. 3**

FIG. 4

515

520

510

200

500

525

**FIG. 5**

600

100 607 101

608

601 602

603 604 605 606

**FIG. 6**

**EUROPEAN SEARCH REPORT**

Application Number

EP 24 18 1014

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/277278 A1 (GORBOVSKOY EVGENY [RU] ET AL) 7 September 2023 (2023-09-07) | 1-12 | INV. G06T13/20 |
| A | * abstract * <br> * paragraph [0003] * | 13-15 | G06T19/20 |
| A | Staten Matthew L. ET AL: "A Comparison of Mesh Morphing Methods for 3D Shape Optimization" In: "Proceedings of the 20th International Meshing Roundtable", 1 January 2011 (2011-01-01), International Meshing Roundtable, XP093221010, ISBN: 978-3-642-24734-7 pages 293-311, DOI: 10.1007/978-3-642-24734-7_16, Retrieved from the Internet: URL:https://www.researchgate.net/profile/Steven-Owen-2/publication/266439748_A_Comparison_of_Mesh_Morphing_Methods_for_3D_Shape_Optimization/links/54f87caa0cf210398e96ac76/A-Comparison-of-Mesh-Morphing-Methods-for-3D-Shape-Optimization.pdf> * abstract * | 1-15 | |
| A | SHU-XIAN ZHENG ET AL: "A novel 3D morphing approach for tooth occlusal surface reconstruction", COMPUTER-AIDED DESIGN, ELSEVIER PUBLISHERS BV., BARKING, GB, vol. 43, no. 3, 9 November 2010 (2010-11-09), pages 293-302, XP028144143, ISSN: 0010-4485, DOI: 10.1016/J.CAD.2010.11.003 [retrieved on 2010-11-29] * abstract * | 1-15 | |

**TECHNICAL FIELDS SEARCHED (IPC)**

G06T

The present search report has been drawn up for all claims

| Place of search | Date of completion of the search | Examiner |
|---|---|---|
| Berlin | 7 November 2024 | Gao, Miao |

EPO FORM 1503 03.82 (P04C01)

## ANNEX TO THE EUROPEAN SEARCH REPORT
## ON EUROPEAN PATENT APPLICATION NO.

EP 24 18 1014

This annex lists the patent family members relating to the patent documents cited in the above-mentioned European search report.
The members are as contained in the European Patent Office EDP file on
The European Patent Office is in no way liable for these particulars which are merely given for the purpose of information.

07-11-2024

| Patent document cited in search report | Publication date | Patent family member(s) | Publication date |
|---|---|---|---|
| US 2023277278 A1 | 07-09-2023 | US 2023277278 A1 | 07-09-2023 |
| | | WO 2023168075 A1 | 07-09-2023 |

EPO FORM P0459

For more details about this annex : see Official Journal of the European Patent Office, No. 12/82